# EUROPÄISCHE PATENTSCHRIFT

(11) **EP 4 039 202 B1**
(45) Veröffentlichungstag und Bekanntmachung des Hinweises auf die Patenterteilung: **18.10.2023**
(21) Anmeldenummer: 22154660.9
(22) Anmeldetag: 02.02.2022
(51) Int. Cl.: A61B 17/22, A61B 17/225, A61B 17/00

(54) **VORRICHTUNG ZUR ERZEUGUNG VON STOSSWELLEN, INSBESONDERE ZUR ERZEUGUNG EINES KOMPRIMIERTEN STOSSWELLENWIRKFELDES**
DEVICE FOR GENERATING SHOCK WAVES, IN PARTICULAR FOR GENERATING A COMPRESSED SHOCK WAVE
DISPOSITIF DE GÉNÉRATION D'ONDES DE CHOC, EN PARTICULIER DE GÉNÉRATION D'UN CHAMP D'ACTION D'ONDE DE CHOC COMPRIMÉ

(30) Priorität: 03.02.2021 DE 202021100542 U
(43) Veröffentlichungstag der Anmeldung: 10.08.2022
(73) Patentinhaber: Stephan, Hugo, 26723 Emden (DE)
(72) Erfinder: Stephan, Hugo, 26723 Emden (DE)
(74) Vertreter: Jabbusch, Matthias

(56) Entgegenhaltungen:
- EP-A1- 2 529 678
- DE-A1-102004 042 895
- US-A- 5 160 336

## Beschreibung

Die Erfindung betrifft eine Vorrichtung zur Erzeugung von Stoßwellen, insbesondere zur Erzeugung eines komprimierten Stoßwellenwirkfeldes mit mindestens einem Stößel zur Erzeugung der Stoßwellen und mit mindestens einem angetriebenen Projektil zum Antreiben des Stößels.

Stoßwellen werden in der Medizin in einer Vielzahl von Anwendungsmöglichkeiten verwendet. Beispielsweise werden die Stoßwellen zur Desintegration von Nierensteinen, zur Behandlung von orthopädischen Indikationen, wie Sehnenansatztendopathien, nicht heilenden Knochenbrüchen und weiteren Indikationen verwendet. Es wird zwischen fokussierten und radialen Stoßwellen unterschieden, wobei die Stoßwellenart je nach Anwendungsfall gewählt werden kann.

Fokussierte Stoßwellen werden auf einen Fokuspunkt fokussiert, so dass die höchste Energiedichte in diesem Fokuspunkt liegt. Fokussierte Stoßwellen können beispielsweise elektromagnetisch oder piezoelektrisch erzeugt werden. Hierbei werden Hochspannungen, beispielsweise zwischen 9 kV und 20 kV, benötigt. Die benötigte hohe Energie macht die Erzeugung von fokussierten Stoßwellen kostenintensiv und störungsanfällig.

Radiale Stoßwellen können mit Hilfe von Druckluft als Energiequelle erzeugt werden. Radiale Stoßwellen breiten sich von der Stoßwellenquelle ausgehend radial aus und weisen ihre höchste Energiedichte nahe der Stoßwellenquelle auf. Hierdurch eignen sich radiale Stoßwellen primär für subkutane, also knapp unter der Haut liegende Indikationen, während fokussierende Stoßwellenquellen auch für tieferliegende Indikationen eingesetzt werden können.

Problematisch bei der Erzeugung von fokussierten Stoßwellen ist, dass für deren Erzeugung eine Hochspannung benötigt wird, wodurch die Erzeugung der Stoßwellen sehr kostenintensiv ist.

Beispielsweise ist in der US 5,160,336 eine Vorrichtung beschriebenen, die einen Stoßwellengenerator des ballistischen Typs umfasst, der ein Projektil aufweist, das dazu vorgesehen ist, eine Hin- und Herbewegung in einem Führungsrohr unter der Steuerung von pneumatischen Mitteln auszuführen, das an einem Ende dieses Führungsrohres angeordnet ist. Ein Wellenleiter weist eine am anderen Ende des Führungsrohrs angeordnete Eintrittsschnittstelle auf, die dazu vorgesehen ist, periodisch von dem Projektil getroffen zu werden und so durch ballistische Wirkung Ultraschallstoßwellen zu erzeugen. Dieser Wellenleiter selbst wird durch einen Metallstab gebildet, dessen eines Ende die oben erwähnte Eintrittsschnittstelle bildet und innerhalb einer Führung längsschwingen kann, während sein anderes Ende dazu vorgesehen ist, durch Schlag auf das zu zerstörende Objekt einzuwirken.

Zu dem betrifft die EP 2 529 678 A1 ein Kombinationsgerät mit zwei unterschiedlichen Druckwellenquellen, von denen eine hohl mit nach außen gerichteter Abstrahlungsrichtung ausgelegt und die andere in dem dadurch verfügbaren Hohlraum angeordnet ist. Zum Beispiel kann eine bekannte ballistische Druckwellenquelle in einem Hohlraum einer ebenfalls bekannten hohlzylindrischen elektromagnetischen Druckwellenquelle mit zugehörigem Reflektor angeordnet werden. Beide Geräte eignen sich zur gleichzeitigen oder direkt kombinierten Behandlung bestimmter Krankheitsbilder.

Die DE 10 2004 042 895 A1 offenbart eine Vorrichtung zum Behandeln von Körpergewebe mit Druck- und/oder Stoßwellen weist einen Behandlungskopf auf, an dessen distalem Ende ein Stößel angeordnet ist, der dazu vorgesehen ist, mit einem distalen Ende eine Druck- und/oder Stoßwelle auf das Körpergewebe abzugeben. Es ist zumindest eine Kappe vorgesehen, die das distale Ende des Stößels abdeckt.

Der Erfindung liegt die Aufgabe zugrunde, eine Vorrichtung zur Erzeugung von Stoßwellen vorzuschlagen, mit der eine kostengünstige Erzeugung von Stoßwellen mit einem komprimierten Stoßwellenwirkungsfeld ermöglicht ist. Die Lösung dieser Aufgabe erfolgt einer Vorrichtung mit den Merkmalen des Patentanspruchs 1 sowie mit einem Verfahren den Merkmalen des Patentanspruchs 10. Weiterbildungen und vorteilhafte Ausgestaltungen sind in den Unteransprüchen angegeben.

Bei einer Vorrichtung zur Erzeugung von Stoßwellen, insbesondere zur Erzeugung eines komprimierten Stoßwellenwirkfeldes mit mindestens einem Stößel zur Erzeugung der Stoßwellen und mit mindestens einem angetriebenen Projektil zum Antreiben des Stößels, wobei der Stößel zumindest abschnittsweise von einem Reflektor umgeben ist, wobei die Innenwandung des Reflektors zur Reflexion der von dem Stößel erzeugten Stoßwellen ausgebildet ist und wobei der Reflektor durch die Innenwandung eines Gehäuses ausgebildet ist und wobei das Gehäuse eine Austrittsöffnung zum Austritt der Stoßwellen aufweist, ist erfindungswesentlich vorgesehen, dass das der Austrittsöffnung des Reflektors zugewandte Ende des Stößels im Wesentlichen kegelförmig ausgebildet ist, und dass die Spitze des im Wesentlichen kegelförmig ausgebildeten Stößels der Austrittsöffnung des Reflektors zugewandt angeordnet ist.

Die Vorrichtung ist zur Erzeugung von Stoßwellen, insbesondere zur Erzeugung eines komprimierten Stoßwellenwirkfeldes ausgebildet, in dem die Stoßwellen ihre höchste Energiedichte aufweisen. Die Stoßwellen werden also auf einen Bereich fokussiert, in dem es zur Überlagerung der Stoßwellen kommen kann. Es handelt sich somit um ein Wirkfeld mit Aufsteilung, wobei eine messbare räumliche Druckerhöhung vorliegt. Das komprimierte Stoßwellenwirkfeld kann hierbei je nach Ausbildung der Vorrichtung unterschiedlich geformt sein, beispielsweise fokussiert oder länglich ausgebildet. Zur Erzeugung der Stoßwellen weist die Vorrichtung einen Stößel auf, der von einem angetriebenen Projektil angetrieben wird. Das Projektil prallt hierbei wiederholt mit einer bestimmten Taktfrequenz auf den Stößel, beziehungsweise auf einen Stoßaufnahmekörper des Stößels, wobei durch den Stößel dann entsprechende Stoßwellen freigesetzt werden. Beispielsweise kann das Projektil im Wesentlichen zylindrisch ausgebildet sein und innerhalb einer Führung, insbesondere entlang eines Führungskanals beweglich gelagert sein, wobei das Projektil in den Führungskanal aufgenommen sein kann. Beispielweise durch einen pneumatischen Antrieb, also beispielsweise durch wiederholt getaktete Luftdruckanstiege, kann das Projektil in der Führung beschleunigt werden, so dass das Projektil getaktet immer wieder auf den Stößel trifft. Beispielsweise kann der Stößel einen Grundkörper aufweisen. Der Grundkörper kann einen Hohlraum, beispielsweise eine Bohrung aufweisen, in die ein Stoßaufnahmekörper aufgenommen werden kann. Der Stoßaufnahmekörper kann beispielsweise im Wesentlichen zylindrisch ausgebildet sein und im in den Hohlraum aufgenommenen Zustand über den Grundkörper überstehen. Der Stoßaufnahmekörper ist dazu vorgesehen, Stöße durch den Aufprall eines Projektils aufzunehmen und an den Stößel zur Erzeugung der Stoßwellen zu übertragen. Hierzu kann der Stoßaufnahmekörper mit einer Führung für das Projektil verbunden sein, so dass das angetriebene Projektil auf den Stoßaufnahmekörper prallt. Die Druckanstiege können beispielsweise mittels einer Druckluftquelle erzeugt werden. Auch andere Antriebsmöglichkeiten des Projektils, beispielsweise durch Magnettechnik oder Ähnliches sind denkbar.

Zumindest das dem Projektil abgewandte Ende des Stößels ist von einem Reflektor, zumindest abschnittsweise, umgeben. Beispielsweise kann das dem Projektil abgewandte Ende des Stößels in einen zumindest abschnittsweise von dem Reflektor umgebenen Hohlraum hineinragen. Vorzugsweise weist der Reflektor mindestens eine Öffnung auf, durch die die mittels des Stößels erzeugten Stoßwellen aus dem Reflektor austreten können. Insbesondere kann der Reflektor durch die Innenwandung eines Gehäuses ausgebildet sein, wobei das Gehäuse die Austrittsöffnung aufweist. Die Austrittsöffnung des Gehäuses wird zur Behandlung vorzugsweise dem zu behandelnden Bereich zugewandt ausgerichtet. Durch das Gehäuse mit seiner Austrittsöffnung, das Projektil sowie dem Stößel kann ein Applikator ausgebildet sein, durch den eine Behandlung mittels Stoßwellen ermöglicht ist.

Der Stößel tritt zumindest abschnittsweise an der der Austrittsöffnung des Reflektors gegenüberliegenden Seite in den Hohlraum des Reflektors ein. Die Innenwandung des Reflektors kann beispielsweise zylindrisch oder auch andersartig, beispielsweise gewölbt, geformt ausgebildet sein. Insbesondere kann die Innenwandung des Gehäuses als ein Reflektor für die von dem Stößel erzeugten Stoßwellen ausgebildet sein. Insbesondere können von dem Reflektor die erzeugten Stoßwellen so reflektiert werden, dass die Stoßwellen aus der Austrittsöffnung austreten und sich in einem Stoßwellenwirkfeld überlagern. Somit herrscht in dem Stoßwellenwirkfeld die höchste Energiedichte, so dass in diesem Wirkfeld eine effektive Behandlung auch in tiefer unter der Haut gelegenen Regionen ermöglicht ist. Das Wirkfeld kann gegenüber fokussierten Stoßwellen vergleichsweise groß ausgebildet sein, so dass eine Behandlung mit dem Stoßwellenwirkfeld ohne Schmerzmittelgabe möglich ist. Durch die Anordnung des Reflektors ist die Ausbildung eines komprimierten Stoßwellenwirkfeldes auch ohne hohen Energieaufwand, wie beispielsweise durch Hochspannung, möglich.

Das Gehäuse umschließt zumindest abschnittsweise einen Hohlraum, durch dessen Innenwandung der Reflektor ausgebildet ist. Das Gehäuse weist eine Austrittsöffnung auf, durch die die mittels des Stößels erzeugten Stoßwellen aus dem Gehäuse austreten können. Vorzugsweise ist die Austrittsöffnung dem Stößel gegenüberliegend angeordnet. Durch das den Reflektor ausbildende Gehäuse mit seiner Austrittsöffnung, den Stößel und das Projektil kann ein Applikator zur Behandlung von verschiedenen medizinischen Indikationen an Mensch oder Tier ausgebildet sein. Hierbei kann die Austrittsöffnung dem zu behandelnden Bereich zugewandt ausgerichtet werden.

Das Ende des Stößels, das der Austrittsöffnung zugewandt ist, also das dem Projektil abgewandte Ende, ist im Wesentlichen kegelförmig ausgebildet, so dass der Stößel eine Spitze ausbildet. Der Stößel kann einen zylinderförmigen Grundkörper aufweisen. Die Außenwandung des zylindrischen Grundkörpers kann parallel zu der Innenwandung des Reflektors geführt sein. Ausgehend von der Außenwandung des zylindrischen Grundkörpers des Stößels erstreckt sich die Kegelfläche, so dass eine Spitze ausgebildet wird. Durch die im Wesentlichen kegelförmig ausgebildete Spitze des Stößels werden Stoßwellen erzeugt, die an der Innenwandung des hohlzylindrisch ausgebildeten Reflektors reflektiert werden und durch die Austrittsöffnung des Reflektors austreten können.

Das der Austrittsöffnung des Reflektors zugewandte Ende des Stößels ist im Wesentlichen kegelförmig ausgebildet, wobei die Spitze des kegelförmigen Endes der Austrittsöffnung des Reflektors zugewandt angeordnet ist. Durch diese Anordnung können die an der Kegelfläche erzeugten Stoßwellen an der Innenwandung, die beispielsweise zumindest abschnittsweise hohlzylindrisch ausgeformt sein kann, des Reflektors reflektiert werden und in einem Wirkfeld gebündelt werden.

In einer bevorzugten Ausführungsform der Erfindung ist der Reflektor zur Reflektion der von dem Stößel erzeugten Stoßwellen in ein Stoßwellenwirkfeld ausgebildet. Die von dem mittels des Projektils angetriebenen Stößel erzeugten Stoßwellen werden an dem Reflektor, also beispielsweise an der Innenwandung des Gehäuses, reflektiert. Die Innenwandung des Gehäuses ist somit als ein Reflektor ausgebildet. Hierbei ist der Reflektor so ausgebildet, dass sich die reflektierten Stoßwellen in einem Bereich überlagern, durch den ein Stoßwellenwirkfeld ausgebildet wird. Das Stoßwellenwirkfeld kann gezielt zur Behandlung verschiedener Indikationen bei sowohl menschlichen als auch tierischen Patienten eingesetzt werden.

In einer Ausführungsform der Erfindung ist das Projektil pneumatisch angetrieben. Beispielsweise kann das Projektil in der dem Projektil zugeordneten Führung pneumatisch, also mit Druckluft, angetrieben werden. Hierzu kann das Projektil beispielsweise durch eine getaktete Luftdruckerhöhung, insbesondere mit einer einstellbaren Frequenz, beschleunigt werden, so dass das Projektil auf den Stößel beziehungsweise den Stoßaufnahmekörper des Stößels trifft. Durch den Stößel werden aufgrund des Aufpralls des Projektils die Stoßwellen erzeugt. Hierzu kann die Führung, insbesondere der Führungskanal des Projektils druckluftleitend, beispielsweise über eine Druckluftleitung, mit einer Vorrichtung zur Erzeugung eines Luftdruckes, beispielsweise einem Kompressor oder Ähnlichem, verbunden sein. Durch die Verwendung eines pneumatischen Antriebes für das Projektil ist eine sehr kostengünstige und energieeffiziente Methode geschaffen, Stoßwellen zu erzeugen.

In einer Ausführungsform der Erfindung ist der Stößel rotationssymmetrisch um eine Symmetrielängsachse ausgebildet, der Stößel ist entlang seiner Symmetrielängsachse beweglich gelagert, die Innenwandung des Reflektors ist rotationssymmetrisch ausgebildet und die Symmetrielängsachse des Stößels und die Symmetrielängsachse der Innenwandung des Reflektors fallen überein. Der Reflektor, insbesondere die Innenwandung des Gehäuses, ist rotationssymmetrisch ausgebildet. Beispielsweise kann die Innenwandung des Reflektors hohlzylindrisch, gewölbt oder andersartig rotationssymmetrisch ausgebildet sein. Insbesondere ist die Innenwandung des Gehäuses rotationssymmetrisch um eine Symmetrielängsachse, bei der es sich um die Zylinderachse bei einem hohlzylindrisch aufgebauten Gehäuse handeln kann, ausgebildet. Ebenso ist der Stößel rotationssymmetrisch um eine Symmetrielängsachse ausgebildet, wobei vorzugsweise die Symmetrielängsachse des Stößels mit der Symmetrielängsachse der Austrittsöffnung und der Innenwandung des Gehäuses übereinstimmt. Durch die Anordnung der Austrittsöffnung, der Innenwandung des Gehäuses und somit des Reflektors sowie des Stößels ist eine effiziente Erzeugung und Überlagerung von Stoßwellen ermöglicht.

In einer Ausführungsform der Erfindung ist die Austrittsöffnung des Gehäuses rotationssymmetrisch ausgebildet und die Symmetrielängsachse der Austrittsöffnung fällt mit den Symmetrielängsachsen des Stößels und der Innenwandung überein. Die Austrittsöffnung des Gehäuses ist rotationssymmetrisch, insbesondere kreisrund, ausgebildet. Die Symmetrielängsachse, um die sich die Austrittsöffnung rotationssymmetrisch erstreckt, fällt mit der Symmetrielängsachse des Stößels überein. Hierdurch ist eine effiziente, gerichtete Abgabe der durch den Stößel erzeugten Stoßwellen aus dem Gehäuse gegeben.

In einer Weiterbildung der Erfindung weisen die Innenwandung des Reflektors und das der Austrittsöffnung des Gehäuses zugewandte Ende des Stößels jeweils eine aneinander angepasste Form auf. Die Innenwandung des Reflektors, also des Gehäuses, die den Reflektor bildet, und die äußere Form des Stößels, insbesondere die Seite des Stößels, die der Austrittsöffnung zugewandt ist und dem Projektil abgewandt ist, weisen eine aneinander angepasste Form auf. Insbesondere ist die äußere Form des Stößels und die Innenseite des Reflektors so aneinander angepasst, dass die von dem Stößel erzeugten Stoßwellen so am Reflektor reflektiert werden, dass die Stoßwellen in einem Stoßwellenwirkfeld zusammengeführt, insbesondere überlagert werden. Insbesondere können die äußere Form des Stößels sowie die Innenseite des Reflektors mittels der Finite-Elemente-Methode berechnet sein. Durch die Anpassung der Form des Stößels und des Reflektors zueinander ist somit eine Erzeugung eines komprimierten Wirkfeldes der Stoßwellen ermöglicht, ohne dass auf Hochspannung oder ähnliche hochenergetische Energiequellen zurückgegriffen werden muss.

In einer Ausführungsform der Erfindung ist der Reflektor zumindest abschnittsweise hohlzylindrisch ausgebildet. Die Innenwandung des Gehäuses, die den Reflektor ausbildet, kann im Wesentlichen hohlzylindrisch, also röhrenförmig ausgebildet sein. Durch ein offenes Ende des Hohlzylinders kann hierbei die Austrittsöffnung des Gehäuses ausgebildet sein. An der der Austrittsöffnung abgewandten Seite des Gehäuses kann der Stößel in das Gehäuse eingefügt sein. Die äußere Wandung des Stößels kann hierbei an der Innenseite des Reflektors geführt sein. Durch das Projektil wird der Stößel getaktet zur Erzeugung von Stoßwellen angeregt, wobei der Stößel parallel zur Innenwandung des Gehäuses bewegt wird. Durch den röhrenförmigen, hohlzylindrischen Aufbau des Reflektors ist eine besonders einfache Konstruktion des Reflektors und der Führung des Stößels ermöglicht.

In einer Weiterbildung der Erfindung ist die Vorrichtung zur Erzeugung eines fokussierten Stoßwellenwirkfeldes ausgebildet und die Kegelfläche des kegelförmig ausgebildeten Endes des Stößels ist konkav in Richtung der Symmetrielängsachse des Stößels gekrümmt. Vorzugsweise ist in dieser Ausführungsform die als Reflektor ausgebildete Innenwandung des Gehäuses hohlzylindrisch ausgebildet. Der Stößel ist rotationssymmetrisch um eine Symmetrielängsachse ausgebildet. Das dem Projektil abgewandte Ende des Stößels ist im Wesentlichen kegelförmig ausgebildet und der Stößel kann einen im Wesentlichen zylinderförmig ausgebildeten Grundkörper aufweisen. Die Kegelfläche des dem Projektil abgewandten Ende des Stößels ist konkav in Richtung der Symmetrielängsachse des Stößels gekrümmt. Die Kegelfläche kann mittels der Finite-Elemente-Methode berechnet sein. Die Kegelfläche weist somit eine Wölbung in Richtung der Symmetrielängsachse des Stößels auf, die Kegelfläche ist also von der Innenwandung des Reflektors weg gewölbt. Die Mantellinie ist also gekrümmt. Die von der konkaven Kegelfläche erzeugten Stoßwellen werden an der Innenwandung des Reflektors reflektiert und können durch die Austrittsöffnung des Gehäuses, also des Reflektors, austreten. Durch die Anordnung der konkaven Kegelflächen zu der zylindrischen Innenwandung des Reflektors werden die Stoßwellen in einem komprimierten Stoßwellenwirkfeld, insbesondere einem fokussierten Wirkfeld überlagert, so dass hier eine erhöhte Energiedichte der Stoßwellen zu erwarten ist.

Alternativ kann die rotationssymmetrisch ausgebildete Innenwandung sich konisch in Richtung der Austrittsöffnung öffnen. Der Innendurchmesser des Reflektors nimmt also von der Seite, an der der Stößel angeordnet ist, ausgehend in Richtung der Austrittsöffnung zu. Beispielsweise kann die Mantellinie des Konus ein bis fünf Grad, insbesondere zwei bis drei Grad zur Symmetrielängsachse des Reflektors, also zur Rotationssymmetrieachse des Reflektors geneigt sein. Entsprechend ist der Stößel mit der konkav in Richtung der Symmetrielängsachse gekrümmten Kegelfläche an die Form des konisch zulaufenden Reflektors angepasst. Durch den konisch zulaufenden Reflektor kann der Fokuspunkt des komprimierten Stoßwellenwirkfeldes im Vergleich zu einem hohlzylindrisch ausgebildeten Reflektor weiter von der Austrittsöffnung entfernt erzeugt werden. Hierdurch sind beispielsweise Behandlungen im tieferliegenden Gewebe eines Patienten ermöglicht.

In einer Ausführungsform der Erfindung ist die Vorrichtung zur Erzeugung eines Stoßwellenwirkfeldes mit länglicher Form ausgebildet und das kegelförmig ausgebildete Ende des Stößels weist eine gerade Mantellinie auf. Vorzugsweise ist in dieser Ausführungsform die als Reflektor ausgebildete Innenwandung des Gehäuses hohlzylindrisch ausgebildet. Das kegelförmige Ende des Stößels, dessen Spitze der Austrittsöffnung zugewandt angeordnet ist, ist vorzugsweise als Kreiskegel, also als Konus, ausgebildet. Es handelt sich um einen geraden Kreiskegel mit einer geraden Mantellinie. Die Mantelfläche, also der Kegelmantel, ist also nicht gewölbt. Durch diese Ausführungsform werden die erzeugten Stoßwellen so an der hohlzylindrisch ausgeführten Innenwandung des Reflektors, also des Gehäuses, reflektiert, dass die Stoßwellen zu einem komprimierten Stoßwellenwirkfeld mit länglicher Form, also durch ein auseinandergezogenes längliches Fokalgebiet, auch Kaustik genannt, zusammengeführt werden. Insbesondere kann hierdurch eine gleichmäßige Energieverteilung entlang des linienartigen Stoßwellenwirkfeldes erreicht werden. Im Vergleich zu einem fokussierten Wirkfeld können hiermit insbesondere reduzierte Spitzendrücke und ein größeres Energiefeld verbunden sein.

In einer Weiterbildung der Erfindung wird durch den Öffnungswinkel des kegelförmig ausgebildeten Endes des Stößels die Länge des länglichen Stoßwellenwirkfeldes bestimmt. Der Öffnungswinkel des Kegels beträgt das doppelte des Winkels zwischen der Mantellinie und der Symmetrieachse eines geraden Kreiskegels. Durch den Öffnungswinkel lässt sich die Länge des linienartigen Stoßwellenwirkfeldes bestimmen. Somit können für verschiedene Einsatzzwecke Stößel mit unterschiedlichen Öffnungswinkeln des Kegels eingesetzt werden um unterschiedlich dimensionierte Stoßwellenwirkfelder zu erreichen.

In einer alternativen Ausführungsform der Erfindung weist die Innenwandung des Reflektors eine gewölbte Form, insbesondere eine halbellipsoide Form auf. Der Reflektor, insbesondere dessen Innenseite kann eine gewölbte Form aufweisen. Hierbei kann die Innenwandung von der Austrittsöffnung ausgehend eine in Richtung der Symmetrielängsachse gerichtete Krümmung in Richtung des Stößels aufweisen. Insbesondere kann der Reflektor an der Austrittsöffnung seinen größten Innendurchmesser aufweisen, wobei der Innendurchmesser sich in Richtung des Stößels verjüngt. Die Innenwandung des Reflektors kann somit beispielsweise im Wesentlichen einen halben Ellipsoid ausbilden. Das dem Projektil abgewandte Ende des Stößels ragt in den halbellipsoiden Hohlraum des Reflektors hinein. Durch das Ende Stößels erzeugte Stoßwellen werden an der halbellipsoiden Innenwandung des Reflektors reflektiert, wobei sich die erzeugten Stoßwellen in einem komprimierten Wirkfeld überlagern. Die gewölbte Innenwandung des Reflektors kann auch parabelförmig oder ähnlich ausgebildet sein.

In einer Weiterbildung der Erfindung ist das der Austrittsöffnung des Reflektors zugewandte Ende des Stößels im Wesentlichen kegelförmig ausgebildet und die Kegelfläche des kegelförmig ausgebildeten Endes des Stößels ist konvex von der Symmetrielängsachse des Stößels weg gekrümmt. Der Stößel kann einen im Wesentlichen zylinderförmigen Grundkörper aufweisen, wobei ein Ende des Stößels dem Projektil zugewandt ist und das andere Ende des Stößels der Austrittsöffnung des Gehäuses zugewandt angeordnet ist. Das dem Projektil abgewandte Ende des Stößels ist im Wesentlichen kegelförmig ausgebildet. Hierbei kann das kegelförmig ausgebildete Ende in den gewölbten, beispielsweise im Wesentlichen halbellipsoiden Hohlraum des Reflektors hineinragen. Die Kegelfläche des kegelförmig ausgebildeten Endes ist konvex von der Symmetrielängsachse des Stößels, insbesondere des zylinderförmigen Grundkörpers, weg gewölbt. Die Kegelfläche weist also eine Wölbung in Richtung der Innenwandung des Reflektors auf. Innerhalb des halbellipsoiden Hohlraumes werden durch das kegelförmige Ende des Stößels, das in den Hohlraum hineinragt, Stoßwellen erzeugt. Die Stoßwellen werden an der Innenseite des Reflektors so reflektiert, dass sie durch die Austrittsöffnung hinaustreten und hier in einem Wirkfeld überlagert werden, so dass hier die höchste Energiedichte zu erwarten ist. Die Kegelfläche des Stößels kann mittels der Finite-Elemente-Methode berechnet werden. Durch die Anordnung des halbellipsoiden Reflektors und des kegelförmigen Stößels, mit dessen konvex gekrümmter Kegelfläche ist eine äußerst effiziente Erzeugung eines Wirkfeldes der Stoßwellen gegeben.

In einer Ausführungsform der Erfindung fallen die Symmetrielängsachse des Stößels und die Symmetrielängsachse des Reflektors überein. Der Stößel kann einen im Wesentlichen zylinderförmigen Grundkörper aufweisen und ist rotationssymmetrisch ausgebildet. Die Innenwandung des Reflektors ist ebenfalls rotationssymmetrisch ausgebildet und kann beispielsweise hohlzylinderförmig oder halbellipsoid ausgeführt sein. Die Symmetrielängsachsen des Stößels sowie des Reflektors können übereinfallen oder sind zumindest parallel zueinander angeordnet. Hierdurch die eine effiziente Erzeugung eines Stoßwellenwirkfeldes ermöglicht.

In einer alternativen Ausführung weist die Vorrichtung mindestens einen Schallbrechungskörper, insbesondere eine akustische Linse, auf, der in dem Hohlraum des Gehäuses zwischen der Austrittsöffnung und dem Stößel angeordnet sein kann. Beispielsweise kann die Innenwandung des Gehäuses hohlzylindrisch ausgebildet sein. Der Stößel kann beispielsweise einen zylindrischen Grundkörper aufweisen und kann abschnittsweise in dem Hohlraum, insbesondere an dem der Austrittsöffnung abgewandten Seite des Hohlraumes, geführt sein. Zwischen dem Stößel und der Austrittsöffnung kann in dem Hohlraum eine akustische Linse ausgebildet sein. Die akustische Linse kann abschnittsweise konkav ausgebildet sein, wobei die akustische Linse jeweils von der Austrittsöffnung und von dem Stößel weg gewölbt ist. Die akustische Linse kann beispielsweise aus einem Kunststoff bestehen. Durch die akustische Linse können Stoßwellen, die von dem Stößel erzeugt werden, gebrochen beziehungsweise gebeugt oder abgelenkt werden und in einem außerhalb des Gehäuses gelegenen Wirkfeld überlagert werden. Das der Austrittsöffnung zugewandte Ende des Stößels kann hierbei flächig, insbesondere ebenmäßig plan ausgebildet sein. Das flächige Ende des Stößels kann eine Ebene aufspannen, die im Wesentlichen parallel zu der von den Rändern der Austrittsöffnung aufgespannten Ebene angeordnet sein kann.

Ein weiterer Aspekt der Erfindung betrifft ein Verfahren zur Erzeugung von Stoßwellen, insbesondere zur Erzeugung eines komprimierten Stoßwellenwirkfeldes mittels eines zumindest abschnittsweise innerhalb eines Reflektors geführten Stößels, wobei der Stößel zur Erzeugung der Stoßwellen mittels eines Projektils in Schwingungen versetzt wird, wobei der Reflektor eine im Wesentlichen hohlzylindrische Innenwandung und eine Austrittsöffnung für die Stoßwellen aufweist, wobei der Stößel ein kegelförmiges Ende aufweist, dessen Spitze der Austrittsöffnung zugewandt angeordnet ist, wobei die von dem kegelförmigen Ende des Stößels erzeugten Stoßwellen von dem Reflektor reflektiert werden und wobei die Stoßwellen mittels des Reflektors auf ein Stoßwellenwirkfeld reflektiert werden.

Zur Erzeugung der Stoßwellen weist die Vorrichtung zur Erzeugung der Stoßwellen einen Stößel auf, der von einem angetriebenen Projektil angetrieben wird. Das Projektil prallt hierbei wiederholt mit einer bestimmten Taktfrequenz auf den Stößel beziehungsweise auf einen Stoßaufnahmekörper des Stößels, wobei durch den Stößel dann entsprechende Stoßwellen freigesetzt werden. Das Projektil kann hierbei durch einen pneumatischen Antrieb angetrieben werden, wobei das Projektil durch wiederholt getaktete Luftdruckanstiege immer wieder beschleunigt wird. Das Projektil trifft somit immer wieder getaktet auf den Stößel beziehungsweise auf einen Stoßaufnahmekörper, der mit dem Stößel so verbunden ist, dass die Stöße durch den Aufprall eines Projektils an den Stößel zur Erzeugung der Stoßwellen übertragen werden. Der Stößel weist ein kegelförmiges Ende auf, das zumindest abschnittsweise in einem Reflektor angeordnet ist. Der Reflektor ist im Wesentlichen hohlzylindrisch ausgebildet. Die von dem zumindest abschnittsweise kegelförmig ausgebildeten Stößel erzeugten Stoßwellen werden in dem Reflektor, also an der hohlzylindrischen Innenwandung, zu einem komprimierten Stoßwellenwirkfeld reflektiert.

In einer Ausführungsform des Verfahrens weist das kegelförmige Ende des Stößels eine gerade Mantellinie auf und die von dem Stößel erzeugten Stoßwellen werden mittels des Reflektors auf ein Stoßwellenwirkfeld mit länglicher Form reflektiert. Das kegelförmige Ende des Stößels ist im Wesentlichen durch einen Kreiskegel ausgebildet, wobei die Mantelfläche nicht gekrümmt ist, der Kegel also eine gerade Mantellinie aufweist. Durch diese Anordnung kann ein linienartiges komprimiertes Stoßwellenwirkfeld erzeugt werden, wobei die höchsten Drücke der Stoßwellen in einem weiteren Bereich erreicht werden, als bei einem fokussierten komprimierten Wirkfeld. Auch sind geringere Spitzendrücke als bei einem fokussierten Wirkfeld zu erwarten.

In einer Ausführungsform des Verfahrens weist das kegelförmige Ende des Stößels eine konkav in Richtung der Symmetrielängsachse des Stößels gekrümmte Mantellinie auf und die von dem Stößel erzeugten Stoßwellen werden von dem Reflektor auf ein fokussiertes Stoßwellenwirkfeld fokussiert. Durch dieses Verfahren kann ein fokussiertes komprimiertes Stoßwellenwirkfeld erzeugt werden, wobei die Stoßwellen in einem engen Bereich fokussiert werden. Je nach angewendeter Norm kann die Größe des fokussierten Stoßwellenwirkfeldes mehrere Millimeter in der Breite und in der Länge betragen und dabei eine Eindringtiefe von beispielsweise über 15 Millimeter erreichen. Durch das fokussierte Stoßwellenwirkfeld können auch tieferliegende Regionen am menschlichen oder tierischen Patienten gezielt behandelt werden.

Im Folgenden wird die Erfindung anhand der in den Figuren dargestellten Ausführungsformen weiter erläutert. Im Einzelnen zeigen die schematischen Darstellungen in:
- Fig. 1:: eine erfindungsgemäße Vorrichtung mit kegelförmigem Stößel und hohlzylindrischem Reflektor;
- Fig. 2:: eine erfindungsgemäße Vorrichtung mit kegelförmigem Stößel und halbellipsoidem Reflektor;
- Fig. 3:: eine Vorrichtung mit Stößel und akustischer Linse;
- Fig. 4:: eine Vorrichtung mit kegelförmigem Stößel zur Erzeugung eines länglichen Stoßwellenwirkfeldes; und
- Fig. 5:: eine Vorrichtung mit kegelförmigem Stößel und konischem Reflektor.

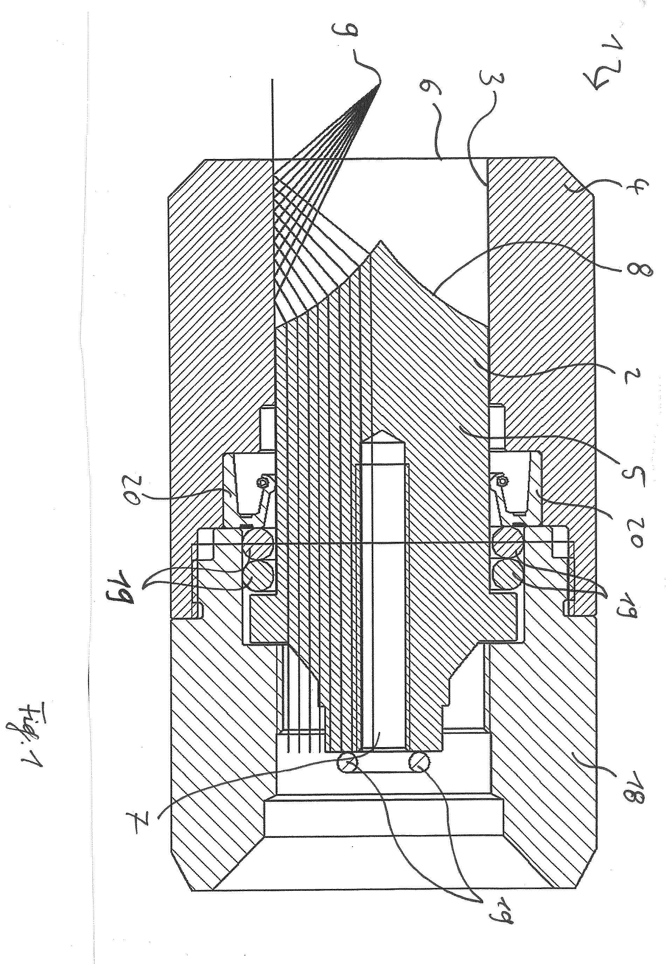

In Fig. 1 ist eine Vorrichtung 1 zur Erzeugung von Stoßwellen mit einem Stößel 2 und einem Reflektor 3 in einer geschnittenen Ansicht dargestellt. Die Innenwandung eines Gehäuses 4 ist hohlzylindrisch ausgebildet, so dass durch die Innenwandung des Gehäuses 4 der Reflektor 3 ausgebildet ist. Der Stößel 2 mit seinem im Wesentlichen zylindrischen Grundkörper 5 ist abschnittsweise in der Innenwandung des Gehäuses 4 geführt. Das Gehäuse 4 weist eine Austrittsöffnung 6 auf, durch die die mittels des Stößels 2 erzeugten Stoßwellen austreten können. Zum Antrieb des Stößels 2, also zu einer Bewegung des Stößels 2 parallel zu der Innenwandung des Gehäuses 4, ist ein pneumatisch angetriebenes Projektil vorgesehen. An der der Austrittsöffnung 6 abgewandten Seite des Stößelgrundkörpers 5 weist dieser einen Hohlraum 7, insbesondere eine Bohrung auf. Dieser Hohlraum 7 ist zur Aufnahme eines Stoßaufnahmekörpers vorgesehen. Der Stoßaufnahmekörper kann beispielsweise zylindrisch ausgebildet sein und im in den Hohlraum 7 aufgenommenen Zustand über den Grundkörper 5 überstehen. Das Projektil trifft getaktet auf den Stoßaufnahmekörper, der die Stoßenergie zur Erzeugung von Stoßwellen auf den Stößel 2 überträgt. An der dem Projektil abgewandten Seite des Stößels 2, also der der Austrittsöffnung 6 zugewandten Seite, ist der Stößel 2 im Wesentlichen kegelförmig ausgebildet. Die Kegelfläche 8 des kegelförmig ausgebildeten Endes des Stößels 2 ist hierbei konkav in Richtung der Symmetrielängsachse des Stößels 2 gekrümmt. Die genaue Ausbildung der Kegelfläche 8 kann hierbei mittels Finite-Elemente-Methode (FEM) berechnet werden. Die Kegelfläche 8 ist hierbei so ausgebildet, dass von ihr erzeugte Stoßwellen an der Innenwandung des Reflektors 3 reflektiert werden und sich in einem Stoßwellenwirkfeld 9 überlagern. Das Stoßwellenwirkfeld 9 ist außerhalb des Gehäuses 4 ausgebildet und stellt die höchste Energiedichte der Stoßwellen dar. Durch das Stoßwellenwirkfeld 9 ist eine zielgerichtete Behandlung von verschiedenen Indikationen möglich. Insbesondere ist durch die spezielle Ausbildung der Kegelfläche 8 eine Erzeugung eines Stoßwellenwirkfeldes 9, insbesondere eines komprimierten Stoßwellenwirkfeldes 9, mit einem pneumatischen Antrieb, also ohne dass auf Hochspannung oder ähnlich hochenergetische Antriebsmethoden zurückgegriffen werden muss, möglich. Das Gehäuse 4 ist zur Ankopplung an ein Kopplungsstück 18 vorgesehen. Das Gehäuse 4 und das Kopplungsstück 18 können über Dichtungen, beziehungsweise 0-Ringe 19 oder Lippendichtungen 20 gegeneinander abgedichtet sein.

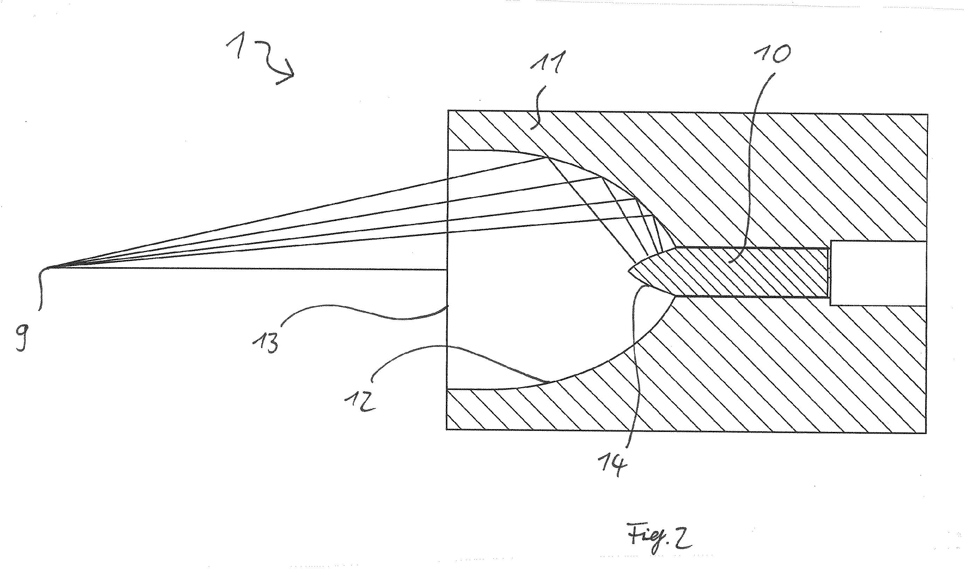

In Fig. 2 ist eine alternative Ausführungsform der Vorrichtung 1 dargestellt. Ein Stößel 10 ist mit seinem einem Projektil abgewandten Ende zumindest abschnittsweise in dem Hohlraum eines Gehäuses 11 angeordnet, wobei die Innenwandung des Gehäuses 11 einen Reflektor 12 ausbildet. Der Reflektor 12 weist eine gewölbte, insbesondere eine halbellipsoide Form auf, wobei der Reflektor 12 an der Austrittsöffnung 13 seinen größten Durchmesser aufweist. Die Kegelfläche 14 des Stößels 10 ist konvex von der Symmetrielängsachse des Stößels 10 weg gewölbt. Die Kegelfläche 14 ist also in Richtung der Wandung des Reflektors 12 gewölbt. Die Kegelfläche 14 kann hierbei mittels der Finite-Elemente-Methode berechnet sein. Die Kegelfläche 14 des Stößels 10 und der Reflektor 12 sind so aufeinander abgestimmt, dass von dem Stößel 10 erzeugte Stoßwellen an der Innenwandung des Reflektors 12 reflektiert werden und in einem Stoßwellenwirkfeld 9 überlagert werden. Somit ist eine Erzeugung eines komprimierten Stoßwellenwirkfeldes 9 möglich, ohne dass auf hochenergetische Antriebsmethoden, wie beispielsweise Hochspannung, zurückgegriffen werden muss.

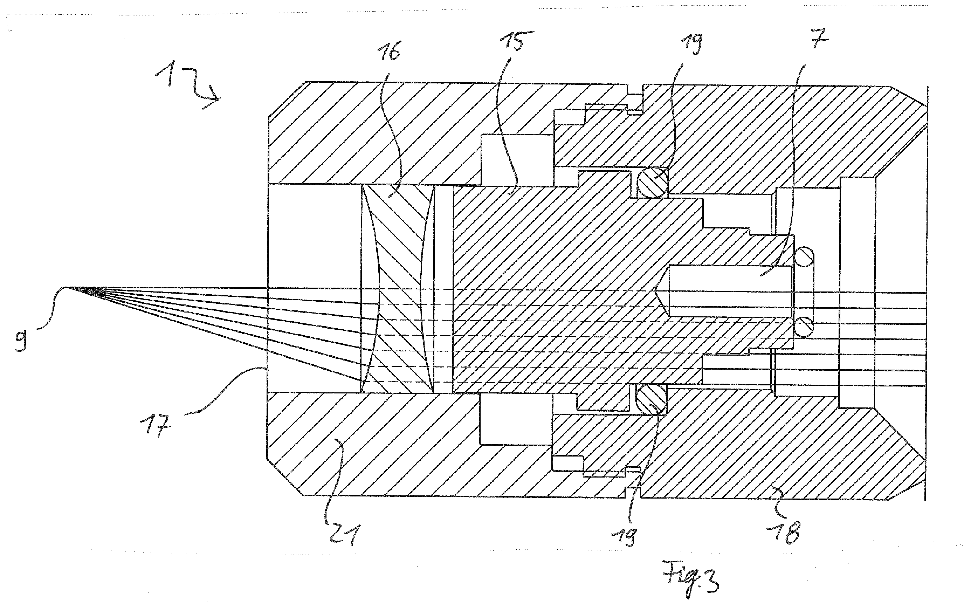

In Fig. 3 ist eine Vorrichtung 1 mit einem Stößel 15 und einer akustischen Linse 16 dargestellt. Das der Austrittsöffnung 17 zugewandte Ende des Stößels 15 ist im Wesentlichen flächig ausgebildet, wobei die von der Fläche aufgespannte Ebene im Wesentlichen parallel zu der von den Rändern der Austrittsöffnung 17 aufgespannten Ebene angeordnet sein kann. Zwischen der Austrittsöffnung 17 und dem Stößel 15 ist eine akustische Linse 16 angeordnet. Die akustische Linse 16 ist im Wesentlichen konkav ausgebildet, so dass die der Austrittsöffnung 17 zugewandte Seite von der Austrittsöffnung 17 weg gewölbt ist und die dem Stößel 15 zugewandte Seite vom Stößel 15 weg gewölbt ist. Es ist ein Hohlraum, insbesondere eine Bohrung zur Aufnahme eines Stoßaufnahmekörpers vorgesehen, wobei das Projektil auf den Stoßaufnahmekörper prallen kann. Vom Stößel 15 erzeugte Stoßwellen werden durch die akustische Linse 16 gebrochen, so dass die abgelenkten Stoßwellen in einem Stoßwellenwirkfeld 9 überlagert werden. Im Stoßwellenwirkfeld 9 ist die höchste Energiedichte zu erwarten. Das Gehäuse 21 ist zur Ankopplung an ein Kopplungsstück 18 vorgesehen. Das Gehäuse 21 und das Kopplungsstück 18 können über Dichtungen 19 gegeneinander abgedichtet sein.

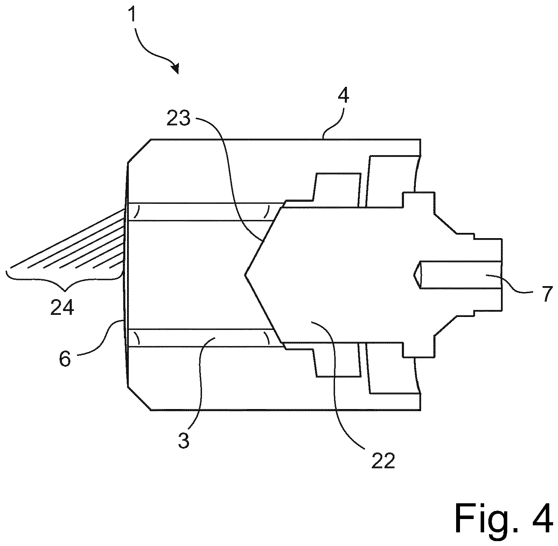

In Fig. 4 ist eine Vorrichtung 1 zur Erzeugung eines länglichen, linienartigen Stoßwellenwirkfeldes 24 dargestellt. Gleiche Bauteile sind mit gleichen Bezugszeichen versehen. In der schematischen Darstellung ist das im Wesentlichen kegelförmig ausgebildete Ende des Stößels 22 durch einen geraden Kreiskegel ausgebildet, wobei die Mantelfläche 23 eine gerade Mantellinie aufweist. Durch den Stößel 22 werden die erzeugten Stoßwellen in einem linienförmigen Stoßwellenwirkfeld 24 komprimiert.

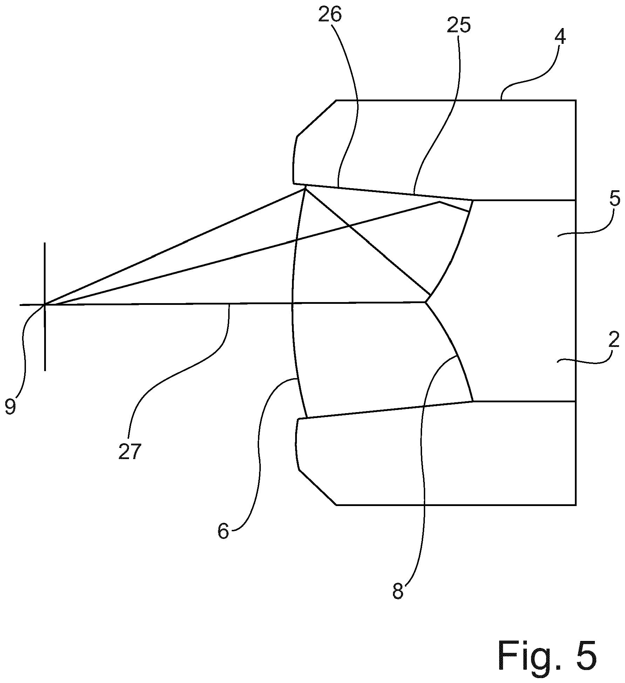

In Fig. 5 ist eine Vorrichtung 1 zur Erzeugung von Stoßwellen mit einem Stößel 2 mit konkav in Richtung der Symmetrielängsachse des Stößels 2 gekrümmten Kegelflächen 8 dargestellt. Der Reflektor 25 ist hierbei konisch ausgebildet, wobei sich der Reflektor 25 in Richtung der Austrittsöffnung 6 weitet. Insbesondere ist die Mantellinie 26 der Innenwandung des Reflektors 25 nicht parallel zur Rotationssymmetrieachse 27 des Reflektors 25 angeordnet, sondern um zwei bis drei Grad zur Rotationssymmetrieachse 27 geneigt. Im Vergleich zu einem hohlzylindrisch ausgebildeten Reflektor kann durch die konische Ausführung des Reflektors 25 der Fokuspunkt des Stoßwellenwirkfeldes 9 weiter von der Austrittsöffnung 6 entfernt erzeugt werden. Der Stößel 2 mit seinen konkaven Mantelflächen kann hierbei an die Innenwandung des Reflektors 25 angepasst sein.

## Patentansprüche

1. Vorrichtung (1) zur Erzeugung von Stoßwellen, insbesondere zur Erzeugung eines komprimierten Stoßwellenwirkfeldes (9, 24), mit mindestens einem Stößel (2, 10, 15, 22) zur Erzeugung der Stoßwellen und mit mindestens einem angetriebenen Projektil zum Antreiben des Stößels (2, 10, 15, 22), wobei der Stößel (2, 10, 15, 22) zumindest abschnittsweise von einem Reflektor (3, 12, 25) umgeben ist, wobei die Innenwandung des Reflektors (3, 12, 25) zur Reflexion der von dem Stößel (2, 10, 15, 22) erzeugten Stoßwellen ausgebildet ist und wobei der Reflektor (3, 12, 25) durch die Innenwandung eines Gehäuses (4, 11, 21) ausgebildet ist und wobei das Gehäuse (4, 11, 21) eine Austrittsöffnung (6, 13) zum Austritt der Stoßwellen aufweist,
**dadurch gekennzeichnet,**
**dass** das der Austrittsöffnung (6, 13) des Reflektors (3) zugewandte Ende des Stößels (2, 22) im Wesentlichen kegelförmig ausgebildet ist, und
**dass** die Spitze des im Wesentlichen kegelförmig ausgebildeten Stößels (2, 10, 22) der Austrittsöffnung (6, 13) des Reflektors (3) zugewandt angeordnet ist.

2. Vorrichtung nach Anspruch 1, **dadurch gekennzeichnet, dass** der Reflektor (3, 12, 25) zur Reflexion der von dem Stößel (2, 10, 15, 22) erzeugten Stoßwellen in ein Stoßwellenwirkfeld (9, 24) ausgebildet ist.

3. Vorrichtung nach einem der Ansprüche 1 oder 2, **dadurch gekennzeichnet, dass** der Stößel (2, 10, 15, 22) rotationssymmetrisch um eine Symmetrielängsachse ausgebildet ist, dass der Stößel (2, 10, 15, 22) entlang seiner Symmetrielängsachse beweglich gelagert ist, dass die Innenwandung des Reflektors (3, 12, 25) rotationssymmetrisch ausgebildet ist und dass die Symmetrielängsachse des Stößels (2, 10, 15, 22) und die Symmetrielängsachse der Innenwandung des Reflektors (3, 12, 25) übereinfallen.

4. Vorrichtung nach einem der Ansprüche 1 bis 3, **dadurch gekennzeichnet, dass** der Reflektor (3, 12, 25) und das der Austrittsöffnung (6, 13) des Reflektors (3, 12, 25) zugewandte Ende des Stößels (2, 10, 22) jeweils eine aneinander angepasste Form aufweisen.

5. Vorrichtung nach einem der Ansprüche 1 bis 4, **dadurch gekennzeichnet, dass** der Reflektor (3) zumindest abschnittsweise hohlzylindrisch ausgebildet ist.

6. Vorrichtung nach einem der Ansprüche 1 bis 5, **dadurch gekennzeichnet, dass** die Vorrichtung zur Erzeugung eines fokussierten Stoßwellenwirkfeldes (9) ausgebildet ist und dass die Kegelfläche (8) des kegelförmig ausgebildeten Endes des Stößels (2) konkav in Richtung der Symmetrielängsachse des Stößels (2) gekrümmt ist.

7. Vorrichtung nach einem der Ansprüche 1 bis 6, **dadurch gekennzeichnet, dass** die Vorrichtung zur Erzeugung eines Stoßwellenwirkfeldes (24) mit länglicher Form ausgebildet ist und dass das kegelförmig ausgebildete Ende des Stößels (22) eine gerade Mantellinie (23) aufweist.

8. Vorrichtung nach einem der Ansprüche 1 bis 4, **dadurch gekennzeichnet, dass** die Innenwandung des Reflektors (12) eine gewölbte Form, insbesondere eine halbellipsoide Form, aufweist.

9. Vorrichtung nach Anspruch 8, **dadurch gekennzeichnet, dass** das der Austrittsöffnung (13) des Reflektors (12) zugewandte Ende des Stößels (10) im Wesentlichen kegelförmig ausgebildet ist und dass die Kegelfläche (14) des kegelförmig ausgebildeten Endes des Stößels (10) konvex von der Symmetrielängsachse des Stößels (10) weg gekrümmt ist.

10. Nicht-chirurgisches und nicht-therapeutisches Verfahren zur Erzeugung von Stoßwellen, insbesondere zur Erzeugung eines komprimierten Stoßwellenwirkfeldes (9, 24) mittels eines zumindest abschnittsweise innerhalb eines Reflektors (3, 12, 25) geführten Stößels (2, 10, 22), wobei der Stößel (2, 10, 22) zur Erzeugung der Stoßwellen mittels eines Projektils in Schwingungen versetzt wird, wobei der Reflektor (3, 12, 25) eine im Wesentlichen hohlzylindrische Innenwandung und eine Austrittsöffnung (6, 13) für die Stoßwellen aufweist, wobei der Stößel (2, 10, 22) ein kegelförmiges Ende aufweist, dessen Spitze der Austrittsöffnung (6, 13) zugewandt angeordnet ist, wobei die von dem kegelförmigen Ende des Stößels (2, 10, 22) erzeugten Stoßwellen von dem Reflektor (3, 12, 25) reflektiert werden und wobei die Stoßwellen mittels des Reflektors (3, 12, 25) auf ein Stoßwellenwirkfeld (9, 24) reflektiert werden.

11. Verfahren nach Anspruch 10, **dadurch gekennzeichnet, dass** das kegelförmige Ende des Stößels (22) eine gerade Mantellinie (23) aufweist und dass die von dem Stößel (22) erzeugten Stoßwellen mittels des Reflektors (3) auf ein längliches Stoßwellenwirkfeld (24) reflektiert werden.

12. Verfahren nach Anspruch 10, **dadurch gekennzeichnet, dass** das kegelförmige Ende des Stößels (2) eine konkav in Richtung der Symmetrielängsachse des Stößels (2) gekrümmte Mantellinie aufweist und dass die von dem Stößel (2, 10) erzeugten Stoßwellen von dem Reflektor (3, 12, 25) auf ein fokussiertes Stoßwellenwirkfeld (9) fokussiert werden.

## Claims

1. A device (1) for generating shock waves, in particular for generating a compressed shock wave effective field (9, 24), having at least one plunger (2, 10, 15, 22) for generating the shock waves and having at least one driven projectile for driving the plunger (2, 10, 15, 22), wherein the plunger (2, 10, 15, 22) is surrounded, at least in sections, by a reflector (3, 12, 25), wherein the inner wall of the reflector (3, 12, 25) is formed to reflect the shock waves generated by the plunger (2, 10, 15, 22), and wherein the reflector (3, 12, 25) is formed by the inner wall of a housing (4, 11, 21), and wherein the housing (4, 11, 21) has an outlet opening (6, 13) for the exit of the shock waves, **characterized in that**
the end of the plunger (2, 22) facing the outlet opening (6, 13) of the reflector (3) is substantially conically shaped, and
**in that** the tip of the substantially conically shaped plunger (2, 10, 22) is arranged facing the outlet opening (6, 13) of the reflector (3).

2. The device according to claim 1, **characterized in that** the reflector (3, 12, 25) is formed to reflect the shock waves generated by the plunger (2, 10, 15, 22) into a shock wave effective field (9, 24).

3. The device according to any one of claims 1 to 2, **characterized in that** the plunger (2, 10, 15, 22) is formed to be rotationally symmetric about a longitudinal axis of symmetry, **in that** the plunger (2, 10, 15, 22) is mounted to be movable along its longitudinal axis of symmetry, **in that** the inner wall of the reflector (3, 12, 25) is formed to be rotationally symmetric, and **in that** the longitudinal axis of symmetry of the plunger (2, 10, 15, 22) and the longitudinal axis of symmetry of the inner wall of the reflector (3, 12, 25) coincide.

4. The device according to any one of claims 1 to 3, **characterized in that** the reflector (3, 12, 25) and the end of the plunger (2, 10, 22) facing the outlet opening (6, 13) of the reflector (3, 12, 25) each have a shape adapted to one another.

5. The device according to any one of claims 1 to 4, **characterized in that** the reflector (3) is formed hollow-cylindrically at least in sections.

6. The device according to any one of claims 1 to 5, **characterized in that** the device is formed for generating a focused shock wave effective field (9) and **in that** the conical surface (8) of the conically shaped end of the plunger (2) is curved concavely in the direction of the longitudinal axis of symmetry of the plunger (2).

7. The device according to any one of claims 1 to 6, **characterized in that** the device for generating a shock wave effective field (24) is formed with an elongated shape and **in that** the conically shaped end of the plunger (22) has a straight generatrix (23).

8. The device according to any one of claims 1 to 4, **characterized in that** the inner wall of the reflector (12) has a curved shape, in particular a semi-ellipsoidal shape.

9. The device according to claim 8, **characterized in that** the end of the plunger (10) facing the outlet opening (13) of the reflector (12) is substantially conically shaped, and **in that** the conical surface (14) of the conically shaped end of the plunger (10) is convexly curved away from the longitudinal axis of symmetry of the plunger (10).

10. A non-surgical and non-therapeutic method for generating shock waves, in particular for generating a compressed shock wave effective field (9, 24) by means of a plunger (2, 10, 22) guided at least in sections within a reflector (3, 12, 25), wherein the plunger (2, 10, 22) is set in oscillation by means of a projectile in order to generate the shock waves, wherein the reflector (3, 12, 25) has a substantially hollow-cylindrical inner wall and an outlet opening (6, 13) for the shock waves, wherein the plunger (2, 10, 22) has a conical end, the tip of which is arranged facing the outlet opening (6, 13), wherein the shock waves generated by the conical end of the plunger (2, 10, 22) are reflected by the reflector (3, 12, 25), and wherein the shock waves are reflected by means of the reflector (3, 12, 25) onto a shock wave effective field (9, 24).

11. The method according to claim 10, **characterized in that** the conical end of the plunger (22) has a straight generatrix (23) and **in that** the shock waves generated by the plunger (22) are reflected by means of the reflector (3) onto an elongated shock wave effective field (24).

12. The method according to claim 10, **characterized in that** the conical end of the plunger (2) has a generatrix concavely curved in the direction of the longitudinal axis of symmetry of the plunger (2), and **in that** the shock waves generated by the plunger (2, 10) are focused by the reflector (3, 12, 25) onto a focused effective shock wave field (9).

## Revendications

1. Dispositif (1) de génération d'ondes de choc, en particulier de génération d'un champ d'action d'ondes de choc (9, 24) comprimé avec au moins un poussoir (2, 10, 15, 22) pour générer des ondes de choc et avec au moins un projectile entraîné pour entraîner le poussoir (2, 10, 15, 22), sachant que le poussoir (2, 10, 15, 22) est entouré au moins par endroits d'un réflecteur (3, 12, 25), sachant que la paroi intérieure du réflecteur (3, 12, 25) est constituée pour la réflexion des ondes de choc générées par le poussoir (2, 10, 15, 22), et sachant que le réflecteur (3, 12, 25) est constitué par la paroi intérieure d'un boîtier (4, 11, 21) et sachant que le boîtier (4, 11, 21) comporte une ouverture de sortie (6, 13) pour la sortie des ondes de choc,
**caractérisé en ce que**
l'extrémité du poussoir (2, 22) tournée vers l'ouverture de sortie (6, 13) du réflecteur (3) est pour l'essentiel de forme conique et
**en ce que** la pointe du poussoir (2, 10, 22) constituée pour l'essentiel de forme conique est disposée tournée vers l'ouverture de sortie (6, 13) du réflecteur (3).

2. Dispositif selon la revendication 1, **caractérisé en ce que** le réflecteur (3, 12, 25) est constitué pour la réflexion des ondes de choc générées par le poussoir (2, 10, 15, 22) dans un champ d'action d'ondes de choc (9,24) .

3. Dispositif selon l'une quelconque des revendications 1 ou 2, **caractérisé en ce que** le poussoir (2, 10, 15, 22) est constitué symétrique en rotation autour d'un axe longitudinal de symétrie, **en ce que** le poussoir (2, 10, 15, 22) est logé de façon mobile le long d'un axe longitudinal de symétrie, **en ce que** la paroi intérieure du réflecteur (3, 12, 25) est constituée symétrique en rotation et **en ce que** l'axe longitudinal de symétrie du poussoir (2, 10, 15, 22) et l'axe longitudinal de symétrie de la paroi intérieure du réflecteur (3, 12, 25) coïncident.

4. Dispositif selon l'une quelconque des revendications 1 à 3,
**caractérisé en ce que** le réflecteur (3, 12, 25) et l'extrémité du poussoir (2, 10, 22) tournée vers l'ouverture de sortie (6, 13) du réflecteur (3, 12, 25) comportent respectivement une forme adaptée l'un à l'autre.

5. Dispositif selon l'une quelconque des revendications 1 à 4,
**caractérisé en ce que** le réflecteur (3) est constitué au moins par endroits de forme cylindrique creuse.

6. Dispositif selon l'une quelconque des revendications 1 à 5, **caractérisé en ce que** le dispositif de génération d'un champ d'action d'ondes de choc (9) concentré est constitué et **en ce que** la surface conique (8) de l'extrémité constituée de forme conique du poussoir (2) est courbée de forme concave en direction de l'axe longitudinal de symétrie du poussoir (2).

7. Dispositif selon l'une quelconque des revendications 1 à 6,
**caractérisé en ce que** le dispositif de génération d'un champ d'action d'ondes de choc (24) est constitué avec une forme longitudinale et **en ce que** l'extrémité constituée de forme conique du poussoir (22) comporte une ligne d'enveloppe droite (23).

8. Dispositif selon l'une quelconque des revendications 1 à 4,
**caractérisé en ce que** la paroi intérieure du réflecteur (12) comporte une forme bombée, en particulier une forme semi-ellipsoïdale.

9. Dispositif selon la revendication 8, **caractérisé en ce que** l'extrémité du poussoir (10) tournée vers l'ouverture de sortie (13) du réflecteur (12) est constituée pour l'essentiel de forme conique et **en ce que** la surface conique (14) de l'extrémité constituée de forme conique du poussoir (10) est courbée de façon convexe en éloignement de l'axe longitudinal de symétrie du poussoir (10).

10. Procédé non chirurgical et non thérapeutique de génération d'ondes de choc, en particulier de génération d'un champ d'action d'ondes de choc comprimé (9, 24) au moyen d'un poussoir (2, 10, 22) guidé au moins par endroits à l'intérieur d'un réflecteur (3, 12, 25), sachant que le poussoir (2, 10, 22) est déporté en oscillations pour générer les ondes de choc au moyen d'un projectile, sachant que le réflecteur (3, 12, 25) comporte pour l'essentiel une paroi intérieure cylindrique creuse et une ouverture de sortie (6, 13) pour les ondes de choc, sachant que le poussoir (2, 10, 22) comporte une extrémité conique dont la pointe est disposée tournée vers l'ouverture de sortie (6, 13), sachant que les ondes de choc générées par l'extrémité conique du poussoir (2, 10, 22) sont réfléchies par le réflecteur (3, 12, 25) et sachant que les ondes de choc sont réfléchies au moyen du réflecteur (3, 12, 25) sur un champ d'action d'ondes de choc (9, 24).

11. Procédé selon la revendication 10, **caractérisé en ce que** l'extrémité conique du poussoir (22) comporte une ligne d'enveloppe droite (23) et **en ce que** les ondes de choc générées par le poussoir (22) sont réfléchies au moyen du réflecteur (3) sur un champ d'action d'onde de choc (24) longitudinal.

12. Procédé selon la revendication 10, **caractérisé en ce que** l'extrémité conique du poussoir (2) comporte une ligne d'enveloppe courbée de façon concave en direction de l'axe longitudinal de symétrie du poussoir (2) et **en ce que** les ondes de choc générées par le poussoir (2, 10) sont concentrées par le réflecteur (3, 12, 25) sur un champ d'action d'ondes de choc (9) concentré.
